Europäisches Patentamt

European Patent Office

Office européen des brevets

(19)

(11) Veröffentlichungsnummer: **0 011 268**
**B1**

(12) # EUROPÄISCHE PATENTSCHRIFT

(45) Veröffentlichungstag der Patentschrift:
**29.12.82**

(21) Anmeldenummer: **79104447.2**

(22) Anmeldetag: **12.11.79**

(51) Int. Cl.³: **A 61 J 3/00**

(54) **Verfahren zur Herstellung von festen Arzneimitteln mit tröpfchenförmig aufgebrachten Wirkstoffen.**

(30) Priorität: **15.11.78 DE 2849494**

(43) Veröffentlichungstag der Anmeldung:
**28.05.80 Patentblatt 80/11**

(45) Bekanntmachung des Hinweises auf die Patenterteilung:
**29.12.82 Patentblatt 82/52**

(84) Benannte Vertragsstaaten:
**AT BE CH DE FR GB IT LU NL SE**

(56) Entgegenhaltungen:
**DE-A-2 656 387**
**SIEMENS-ZEITSCHRIFT Band 51, Nr. 4, 1977**
**Berlin J. HEINZL et al. »Lautloser Tintendruck für**
**Schreibstationen« Seiten 219 bis 221**

(73) Patentinhaber: **Dr. Karl Thomae GmbH, Postfach 720,**
**D-7950 Biberach (Riss) (DE)**

(72) Erfinder: **Voss, Gunther M., Ziegelstadel 10,**
**D-8918 Diessen (DE)**
Erfinder: **Gruber, Peter, Dr. Dipl.-Chem.,**
**Wetterkreuzstrasse 36, D-7950 Biberach 1 (DE)**

## Verfahren zur Herstellung von festen Arzneimitteln mit tröpfchenförmig aufgebrachten Wirkstoffen

Die Erfindung betrifft Verfahren zur Herstellung von festen Arzneimitteln mit tröpfchenförmig aufgebrachten Wirkstoffen. Die Arzneimittel werden durch dosiertes Bepunkten mit flüssigen, gelösten oder suspendierten Wirkstoffen auf die entsprechenden Arzneiformträger mittels piezoelektrischer Dosiersysteme hergestellt.

Die pharmazeutische Industrie ist in den letzten Jahren durch intensive Forschung zu immer effektiveren Wirkstoffen gelangt. Während die meisten in früheren Jahren entwickelten Wirkstoffe im mg-Bereich dosiert wurden (übliche Dosen: ca. 20 – 150 mg pro Tablette), sind inzwischen Wirkstoffe erfunden worden, deren Dosis pro Tablette bei wenigen $\gamma$ (1 $\gamma$ = 0,001 mg) liegt. Derartige Wirkstoffe bereiten bei der Verarbeitung Schwierigkeiten, da die Menge des Wirkstoffs im Verhältnis zur Restmasse des Formlings unverhältnismäßig klein wird. Aus diesem Grund kann durch Vermischen keine ausreichend gleichmäßige Verteilung des Wirkstoffs im gesamten Formling erreicht werden. So wurden z. B. bei einer Untersuchung der auf dem US-Markt befindlichen Digoxintabletten Abweichungen von bis ±50% von der deklarierten Menge Digoxin je Tablette festgestellt.

Bisher mußte der Wirkstoff in dem Granulat gleichmäßig verteilt werden, dabei sind bei niedrig zu dosierenden Wirkstoffen umfangreiche Sicherheitsvorkehrungen zu treffen, beispielsweise auch zum Schutze des Bedienungspersonals; des weiteren sind umfangreiche technische Operationen, wie Mischen, Granulieren, Verreiben bzw. Feinstmahlen, unumgänglich notwendig. Im übrigen kann beim Verpressen zu Tabletten von niedrigdosierten Wirkstoffen durch nichtvermeidbare Sintervorgänge die Auflösegeschwindigkeit und damit die Resorbierbarkeit des Wirkstoffes im Körper negativ beeinflußt werden. Bei der Herstellung von Tabletten oder Drageekernen mit sehr niedrig zu dosierenden Wirkstoffen kommt es ganz besonders auf die exakte Dosierung dieser Wirkstoffe an, diese geforderte exakte Dosierung ist aber den den herkömmlichen Verfahren oft nur unzureichend möglich.

Um von der vorstehend beschriebenen konventionellen Methode wegzukommen, wurde vorgeschlagen (DE-A 1-2 656 387), den aktiven Bestandteil auf ein als Wirkstoffträger dienendes Band in nasser oder trockener Form aufzubringen. Für die Aufbringung der aktiven Bestandteile in flüssiger Form wurde eine elektrostatische Sprühstrahlablagerung besonders empfohlen, alternativ aber auch das Eintauchen des Trägermaterials in ein Bad mit Sättigungsflüssigkeit. Bei dem Verfahren der elektrostatischen Sprühstrahlablagerung wird eine vorher abgemessene Menge der Lösung oder Suspension, die den aktiven Bestandteil enthält, einer Vorrichtung zugeführt, die einen Strahl von Mikrotröpfchen versprüht; die Mikrotröpfchen werden auf eine spezielle Fläche des Bandes unter Zuhilfenahme eines elektrostatischen Feldes konzentriert. Die Tröpfchen werden nach dem Prinzip der allgemein bekannten Staubabscheidung auf dem Arzneiformträger durch elektrostatische Anziehung niedergeschlagen, wobei eine statistische Verteilung der einzelnen Tröpfchen verschiedener Tröpfchengröße auf einer Fläche des Arzneiformträgers erzielt wird.

Eine exakte Dosierung des Wirkstoffes bei einem möglichst punktförmigen Auftragen desselben auf den Wirkstoffträger ließ sich bis heute nur mit einer Dosiervorrichtung in Form einer Pipette durchführen. Mit Hilfe einer Pipette wird die Lösung oder Suspension in diskrete Tröpfchen definierten Volumens aufgeteilt, damit wird es möglich, eine definierte Menge des Wirkstoffes auf einen Arzneiformträger zu bringen. Eine rationelle Serienfertigung ist mit dieser althergebrachten Methode natürlich nicht möglich.

Die Bildung von Codierungen oder von Schriftzeichen mittels piezoelektrischer Dosiersysteme wird von J. Heinzl et al. in dem Artikel »Lautloser Tintendruck für Schreibstationen« auf den Seiten 219 bis 221 der Siemens-Zeitschrift, Band 51, Heft 4, 1977, Berlin, beschrieben. Es handelt sich hierbei um die Beschriftung von Papiermaterialien; von der Anwendung einer entsprechend abgeänderten Technik auf das Auftragen bestimmter Wirkstoffmengen auf Arzneiformträger, beispielsweise auf Tabletten oder Dragées, mit ihren besonderen Oberflächenverhältnissen war in diesem Artikel nirgends die Rede.

Es bestand die Aufgabe, eine definierte Anzahl von Tröpfchen definierten Volumens einer einen Wirkstoff in bekannter Konzentration enthaltenden Lösung oder Suspension auf einen Punkt oder eine beschränkte Fläche eines Wirkstoffträgers, gegebenenfalls aber auch nach einer vorbestimmten geometrischen Verteilung, aufzubringen; eine Lösung dieser Aufgabe, die eine rationelle Massenfertigung gestattet, war durch die obengenannten Publikationen nicht nahegelegt worden.

Es wurde nun gefunden, daß eine extrem genaue Dosierung solcher Wirkstoffe auf Arzneiformträger dadurch erreicht werden kann, daß der flüssige, gelöste oder suspendierte Wirkstoff in definierter Menge in Form von diskreten Tröpfchen von definiertem Volumen auf den Arzneiformträger aufgepunktet wird. Das Aufpunkten geschieht mittels z. B. röhrenförmiger oder plättchenförmiger piezoelektrischer Dosiersysteme.

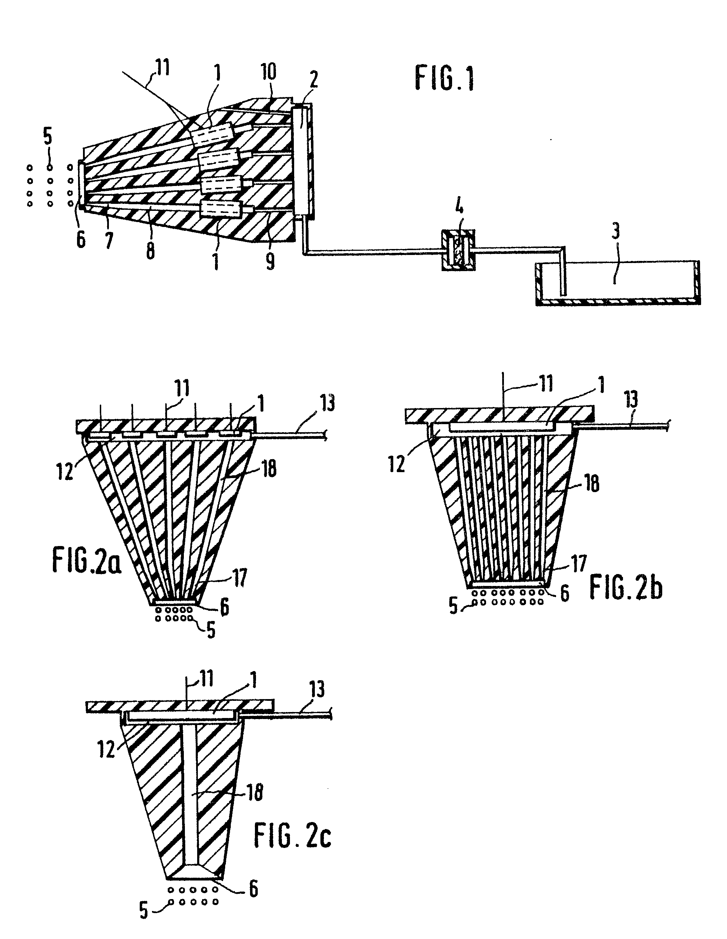

Ein zum Aufpunkten des flüssigen, gelösten oder suspendierten Wirkstoffes auf den Arzneiformträger geeignetes System besteht beispielsweise aus einem oder einer ganzen Reihe von Kanälen in der Art, daß ein röhrenförmiger Piezoschwinger einen Abschnitt jeden Kanals konzentrisch umhüllt. Als Elektroden zum Anlegen des elektrischen Feldes dienen leitfähige Schichten, z. B. Silberschichten, auf den Mantelflächen des röhrenförmigen Piezoschwingers. Die Austrittsöffnungen der Kanäle sind düsenförmig ausgebildet und werden so ausgerichtet, daß jede einzelne einen bestimmten Bereich des Arzneiformträgers mit einem oder einer definierten Anzahl, volumenmäßig definierter Tröpfchen des flüssigen, gelösten oder suspendierten Wirkstoffes bepunktet. Die einzelnen Kanäle sind in ihrer Aufgabeseite z. B. an eine gemeinsame Verteilerplatte, die in Verbindung mit einem Vorratsbehälter steht, angeschlossen und werden von dort mit dem flüssigen oder suspendierten Wirkstoff versorgt (vgl. Fig. 1).

Das Zurückfließen der Flüssigkeit oder Suspension in dem Düsenkanal ist beispielsweise dadurch erschwert, daß der Düsenkanal zur Austrittsöffnung hin verengt ist. Infolge der Eigenschaft von Piezoschwingern, z. B. von piezokeramischen Massen, beim Anlegen eines bestimmten elektrischen Feldes eine elastische Deformation zu erleiden, entsteht in den röhrenförmigen Piezoschwingern eine auf die Flüssigkeit gerichtete Stoßwelle. Die damit verbundene Druckerhöhung führt zum Herausschießen kleinster Wirkstoffmengen in Keulenform aus den Austrittsöffnungen, wobei diese Flüssigkeitskeulen nach Verlassen der Austrittsöffnungen Kugelform annehmen. Der Durchmesser eines Kanals beträgt vorteilhafterweise circa 1 mm in seinem Mittelteil, der einzelne Kanal ist an seiner Austrittsöffnung verjüngt. Der Durchmesser der Austrittsöffnung beträgt beispielsweise 0,1 mm.

Der Vorratsbehälter liegt tiefer als die Austrittsöffnungen, man spricht deshalb von einem Unterdrucksystem. Durch den Höhenunterschied entsteht ein statischer Unterdruck in den Kanälen. Dieser statische Unterdruck wird beim Anlegen des elektrischen Feldes in Verbindung mit der Kapillarwirkung in den Kanälen für einen kurzen Augenblick überkompensiert.

Der Kanal, der von dem Piezoschwinger umschlossen wird, kann vor oder hinter diesem beliebig gekrümmt sein; diese Ausgestaltung dient zur besseren Anpassung des Wirkstoff-Dosiersystems an die räumlichen Gegebenenheiten z. B. der Tablettenpresse. Der Kanal kann jedoch auch räumlich nach dem Piezoschwinger sich in zwei oder mehrere Kanäle verzweigen, so daß ein Piezoschwinger mehrere Kanäle mit separaten Austrittsöffnungen versorgt.

Die Austrittsöffnungen können z. B. Löcher in einer Glas- oder Metallplatte sein. Besteht der Kanal aus einer Glaskapillare, so kann die Austrittsöffnung durch das Ausziehen des Glasröhrchens an seinem Ende gebildet werden.

Eine andere vorteilhafte Ausführungsform zum Bepunkten mit flüssigen oder suspendierten Wirkstoffen besteht in der Verwendung von plättchenförmigen bzw. planaren, nach dem piezoelektrischen Prinzip arbeitenden Wandlern, die vorzugsweise in einer Verteilerkammer konzentrisch über dem Eingang der Kanäle angebracht sind; am Ende der Kanäle befinden sich wieder verjüngte Austrittsöffnungen. Bei einer vorzugsweisen Ausführungsform liegt das Piezoplättchen in einer Verteilerkammer horizontal konzentrisch zum vertikal wegführenden Kanal. Die Piezoplättchen liegen in oder an dieser Kammer zur Aufnahme des flüssigen, gelösten oder suspendierten Wirkstoffes. Es können auch mehrere Kanäle von einer gemeinsamen Kammer wegführen, die ihrerseits an einer gemeinsamen Flüssigkeitsversorgung angeschlossen ist. So kann z. B. auch ein planarer Schwinger (Piezoplättchen) gleichzeitig eine Druckwelle in mehreren, sich an die gleiche Verteilerkammer anschließenden Kanälen erzeugen.

Eine weitere vorteilhafte, konstruktiv vereinfachte Ausführungsform beinhaltet einen hubstarken, planaren Schwinger in der Kammer und einen von der Kammer ausgehenden Kanal, an dessen Ende sich mehrere, gegebenenfalls räumlich verschieden ausgerichtete Düsen befinden. Durch eine derartige Anordnung kann mit einem einzigen, von dem Piezoschwinger erzeugten Hub eine Flächenbepunktung des Arzneimittelformlings erreicht werden (vgl. Fig. 2a, b, c).

Um die durch Piezowandler erzeugten Tröpfchen gezielt an die gewünschten Oberflächen der Arzneiformträger zu bringen, ist es in manchen Fällen von Vorteil, diese nach Verlassen der Austrittsöffnungen durch Anlegen einer elektrischen Spannung aufzuladen, um sie anschließend durch elektrostatische Ablenkung in ihrer Bahn gezielt zu steuern. Diese Steuerung läßt sich mit gebräuchlichen Mitteln durchführen, beispielsweise nach dem Prinzip der Kathodenstrahlablenkung in einer Fernsehröhre.

Die piezokeramischen Körper können auch als Ventile zum Einsatz kommen, wenn man die Wirkstoffflüssigkeit oder -suspension mit Druck dem Schwinger bzw. Wandler zuführt, der sich je nach Ansteuerung öffnet oder schließt. Beim Ansteuern öffnet sich kurzfristig beispielsweise eine schlitzförmige Öffnung in einem, die Flüssigkeit unter Druck enthaltenden Kanal, durch die das Füllgut in Tropfenform abgegeben wird; die Öffnung kann in dem Schwinger selbst, der als Ventil den unter Druck stehenden Raum schließt, oder in der Randzone zwischen dem Schwinger und dem die Wände des Kanals bildenden Material angebracht sein. Dieser Vorgang ist auch in umgekehrter Weise möglich, bei Ansteuerung verschließt der Schwinger den unter Druck stehenden Raum.

Das bevorzugt eingesetzte Wirkstoffdosiersystem, auch Mikropumpensystem genannt, arbeitet mit röhrchenförmigen oder plättchenförmigen Piezoschwingern. Beim Anlegen eines Spannungsimpulses von beispielsweise 100 Volt und einer Impulsdauer von 20 Mikrosekunden werden Tröpfchen mit einer Geschwindigkeit von ca. 4 m/sec und sehr konstantem Tröpfchengewicht von beispielsweise 0,8 μg (0,0008 mg) ausgestoßen. Je nach elektronischer Ansteuerung kann die Tropfenfrequenz zwischen 1 und 50 000 Tropfen pro Sekunde, bevorzugt bei 3000 Tropfen pro Sekunde, liegen.

Die Dosierung läßt sich durch folgende Parameter steuern:

a) durch den Durchmesser der Austrittsöffnung der Düsenkanäle,
b) durch die an den Piezoschwinger angelegte Spannung,
c) durch die Tröpfchenfrequenz,
d) durch die Anzahl der Düsenkanäle,
e) durch die Hubstärke des verwendeten röhrenförmigen oder planaren Schwingers,
f) durch die Wirkstoffkonzentration der Lösung bzw. Suspension,
g) durch die Anzahl der Wirkstoffpunkte pro Arzneiformträger.

Die Fig. 1 und 2 sollen in beispielhafter Weise schematisch einige mögliche Vorrichtungen zum Bepunkten von Arzneiformträgern mit flüssigen, gelösten oder suspendierten Wirkstoffen verdeutlichen.

Die Fig. 1 zeigt schematisch im Querschnitt ein Bepunktungssystem mit piezoelektrischen Wandlern (1), die jeweils einen Düsenkanal (8) umhüllen; der Düsenkanal endet in einer Verjüngung (7); die einzelnen Verjüngungen (7) stehen an entsprechenden Öffnungen einer Austrittsdüsenplatte (6) an, wobei die durch die Verjüngungen (7) und die Öffnungen der Austrittsplatte (6) gebildeten Düsen bei Betätigung der Vorrichtung Flüssigkeitströpfchen (5) abgeben. Der Düsenkanal (8) ist über einen verjüngten Flüssigkeitskanal (9) an eine Flüssigkeitskammer (2) angeschlossen. Die Verteilerkammer (2) weist einen Entlüftungskanal (10) auf; die Verteilerkammer ist über eine Filterplatte (4) mit einem Flüssigkeitsvorratsbehälter (3) verbunden. Die elektrische Ansteuerung der piezoelektrischen Wandler erfolgt über Kontakte (11).

Die Fig. 2a, 2b und 2c zeigen Querschnitte durch verschieden konstruierte Bepunktungsköpfe mit planaren, nach dem piezoelektrischen Prinzip arbeitenden Wandlern. Hierbei ist (1) ein planarer piezoelektrischer Wandler mit Kontakten (11) zur elektrischen Ansteuerung. Der planare piezoelektrische Wandler liegt in einer Flüssigkeitskammer (12), die über die Flüssigkeitsleitung (13) mit einem Vorratsbehälter verbunden ist. Von der Kammer (12) geht ein oder mehrere Düsenkanäle (18) ab, deren Verjüngungen (17) an einer Austrittsdüsenplatte (6) enden; (5) stellen die freigesetzten Flüssigkeitströpfchen dar.

Zur dosierten Aufbringung von Wirkstoffen auf Arzneiformträger ergeben sich damit folgende Möglichkeiten:

Bei der Herstellung von Tabletten geht man von einem Trägergranulat aus, welches in üblicher Weise über den Granulatfüllschuh in die Matrizen volumetrisch eingebracht wird. Unmittelbar nach Verlassen der Füllstation wird die Wirkstoffflüssigkeit auf das Granulat eingepunktet. Verwendet man dabei ein Dosiersystem (vgl. Fig. 2c) mit einem hubstarken Planarschwinger, das eine an die Form und Größe der Oberfläche der Matrize angepaßte Anzahl von Düsenöffnungen aufweist, so kann z. B. durch einen einzigen Hub die gewünschte Wirkstoffmenge verteilt aufgepunktet werden.

Eine gleichmäßige Bepunktung der Oberfläche des in der Matrize befindlichen Granulates kann auch dadurch erfolgen, daß die in einer Reihe angeordneten Mikropumpen elektronisch so angesteuert werden, daß nur die Mikropumpen Wirkstoffflüssigkeit abgeben, die gerade über der vorbeilaufenden Granulatoberfläche sich befinden.

Die oben beschriebenen Bepunktungssysteme können aber auch an der Stelle in der Tablettenpresse angebracht sein, an der der fertige Preßling zum Ausstoß aus der Matrizenform ansteht, es wird also der flüssige Wirkstoff auf den fertigen Formling direkt aufgepunktet. Schließlich kann ein Bepunkten des Wirkstoffträgers durch diese beiden Bepunktungssysteme auch in der Weise durchgeführt werden, daß die fertigen Placebo-Wirkstoffträger an den genannten Dosiersystemen außerhalb des Bereiches der Tablettiermaschine vorbeigeführt werden; mit anderen Worten, die Formlinge werden vereinzelt und in Reihen geordnet an dem Bepunktungssystem vorbeigeführt, wobei z. B. durch Fotozellen die Bepunktungsvorgänge ausgelöst werden. Die erwähnten Dosiersysteme können natürlich auch an Kapselabfüllmaschinen angebracht werden.

Bei flächigen, konkaven oder konvexen Wirkstoffträgern, z. B. Oblaten, lassen sich die einzelnen Mikropumpen so ansteuern, daß dadurch auf der Fläche des Wirkstoffträgers ein geometrisches Muster entsteht; wird die Wirkstofflösung angefärbt, so kann gleichzeitig mit der Wirkstoffdotierung auch eine Codierung oder Beschriftung auf der Trägerfläche berührungslos erzielt werden. Da der Dotierungsvorgang berührungslos abläuft, spielt die eigentliche Geometrie der Oberfläche des Wirkstoffträgers keine Rolle, diese Oberfläche kann z. B. konvex oder konkav oder auch völlig unregelmäßig geformt sein. Die Verwendung einer eingefärbten Wirkstofflösung bietet noch einen zusätzlichen Vorteil: mit Hilfe eines automatischen Lesegerätes (z. B. Scanner) kann die Vollständigkeit der Dosierung geprüft werden.

Die abgegebenen Tröpfchen können natürlich auch durch ein elektronisches Zählwerk erfaßt werden. Der Wirkstoff wird im allgemeinen in physiologisch unbedenklichen Lösungsmitteln, wie Wasser, Glycerin, Glycol oder Alkoholen, wie Äthanol, gelöst. Der Wirkstoff kann auch in einer Kugelmühle fein vermahlen in einem Suspensionsmittel suspendiert werden.

Es werden bei den vorstehend beschriebenen Bepunktungssystemen jeweils Tröpfchen der Wirkstofflösung oder -suspension von genau gleicher Größe und gleichem Gewicht auf den Wirkstoffträger übertragen; hierbei kann die Zahl der pro Bepunktungsvorgang aufgebrachten Tröpfchen durch eine elektronische Steuerung, gewünschtenfalls jeder einzelnen Düse, genau begrenzt werden, wobei bei vorgegebenem Gehalt des Wirkstoffes z. B. in der Lösung oder Suspension eine außerordentlich genaue Dosierung ermöglicht wird. Die einzelnen Bepunktungsvorgänge sind selbstverständlich mit der Drehzahl der Tablettiermaschine verkoppelt; zur Erzeugung entsprechender Signale, die der Steuerung eingegeben werden, dienen z. B. die Matrize abtastende Fotozellen.

Sehr vorteilhaft für die Herstellung bestimmter Arzneimittelformen wirkt sich die Tatsache aus, daß durch ein gezieltes Bepunkten, insbesondere bei flächigen Wirkstoffträgern, sich einzelne Zonen mit ganz bestimmten Arzneimittelkonzentrationen, wobei diese Konzentrationen von Träger zu Träger in bestimmten Verhältnissen auch voneinander abweichen können, dotieren lassen. So kann es in manchen Fällen vorteilhaft sein, wenn der flüssige, gelöste oder suspendierte Wirkstoff auf dafür vorgesehene Zonen eines flächigen Wirkstoffträgers auch in voneinander verschiedenen Dosierungen aufgepunktet wird.

Das Verfahren der dosierten Bepunktung von Arzneiformträgern eröffnet eine Möglichkeit zur exakten Wirkstoffdotierung, wie sie nach den herkömmlichen Methoden nicht erreichbar war. Beim Einsatz von Placebo-Tabletten oder Dragées können diese Placebo-Wirkstoffträger aus billigen Wirkstoffen rationell und damit wirtschaftlich in großen Mengen hergestellt werden. Anstelle von Placebo-Tabletten oder Dragées können auch flächige Arzneiformträger, wie Oblaten, Gelatineplatten oder solche aus saugfähigen Stoffen verwendet werden; nach den herkömmlichen Dotierungsmethoden lassen sich diese Formträger nicht in wirtschaftlicher Weise exakt beaufschlagen. Bei Einsatz der vorstehend erwähnten Träger entfallen aufwendige Operationen, wie Mischen, Granulieren, Trocknen und Verpressen von Granulaten. Damit ist eine wesentliche Reduzierung des Maschinenparkes und der Produktionsflächen möglich, und das fertige Arzneimittel kann billiger produziert werden.

Die folgenden Beispiele erläutern die Erfindung, ohne sie aber zu begrenzen:

Beispiel 1

Tabletten

| Milchzucker | 75 mg |
|---|---|
| Maisstärke | 125 mg |
| sek. Calciumphosphat | 40 mg |
| lösliche Stärke | 3 mg |
| Magnesiumstearat | 4 mg |
| kolloidale Kieselsäure | 3 mg |
| | 250 mg |

Ein Teil des vorstehend beschriebenen Gemisches wird intensiv mit einer wäßrigen Lösung der löslichen Stärke durchgeknetet und üblicherweise mit Hilfe eines Siebes granuliert. Das Granulat wird mit den restlichen Hilfsstoffen vermischt und zu Tabletten von je 250 mg Gewicht verpreßt. Diesen Tabletten wird dann mit Hilfe eines planaren Schwingers, der in einer Kammer über einem, an seinem anderen Ende mit einem Düsenkranz versehenen Kanal angebracht ist, im Moment des Austritts aus der Preßkammer eine Dosis von 0,06 mg des (gelösten) Wirkstoffs (z. B. Clonidin-Hydrochlorid in Wasser/Äthanol) aufgepunktet. Bei der Wirkstoffbestimmung an 20 einzelnen Tabletten lagen alle Werte innerhalb der Fehlerbreite der Analysenmethode ($\pm 0,5\%$).

Beispiel 2

Ein eßbarer Arzneiformträger wird mit dem Dosiersystem, ausgestattet mit 12 röhrenförmigen Piezoschwingern, bedruckt. Das Schriftbild, das Präparatename, Dosierung und Einnahmezeit umfaßt, setzt sich aus 250 Punkten zusammen (1 Buchstabe wird aus ca. 20 Punkten gebildet). Das Gewicht eines Tropfens beträgt ca. $1\,\gamma = 0,001$ mg. Die Konzentration der Wirkstofftinte ist so eingestellt, daß das Schriftbild exakt $100\,\gamma = 0,1$ mg Wirkstoff enthält. Das Dosiersystem arbeitet mit einer Geschwindigkeit von 300 Buchstaben pro Sekunde, die Wirkstofftropfenfrequenz beträgt 3000 Punkte pro Sekunde.

Beispiel 3

In einer Vereinzelungsapparatur werden aus Milchzucker, Maisstärke und mikrokristalliner Cellulose hergestellte 9 mm Placebotabletten mit einer konstanten Geschwindigkeit von 1 m pro Sekunde an einem Dosierkopf vorbeigeführt. Der Dosierkopf besteht aus einem kräftigen planaren Piezoschwinger. 100 Düsenkanäle sind kreisförmig so angeordnet, daß sie gleichmäßig die gesamte Oberfläche der Tablette bepunkten. Der Dosiervorgang ist in einer Millisekunde beendet. Während dieser Zeit hat der planare Piezoschwinger 5 Hübe durchgeführt und insgesamt 5 mg einer 20%igen Wirkstoffsuspension abgegeben.

Bei der Wirkstoffbestimmung an 20 einzelnen Tabletten lagen alle Werte innerhalb der Fehlerbreite der Analysenmethode ($\pm 1\%$). Es können ca. 200 000 Tabletten/h mit der Wirkstoffsuspension bepunktet werden.

**Patentansprüche**

1. Verfahren zur Herstellung von festen Arzneimitteln, bei dem flüssige, gelöste oder suspendierte Wirkstoffe mittels einer Dosiervorrichtung in diskrete Tröpfchen definierten Volumens aufgeteilt und in definierter Anzahl auf einen Arzneiformträger aufgebracht werden, dadurch gekennzeichnet, daß die Wirkstoffe mittels piezoelektrischer Dosiersysteme aufgepunktet werden.

2. Verfahren nach Anspruch 1, dadurch gekennzeichnet, daß der flüssige, gelöste oder suspendierte Wirkstoff vor dem Verpressen des als Arzneiformträger dienenden Granulats zu Tabletten oder Kernen in dieses eingepunktet wird.

3. Verfahren nach Anspruch 1, dadurch gekennzeichnet, daß der flüssige, gelöste oder suspendierte Wirkstoff nach dem Preßvorgang auf den dabei gewonnenen Formling aufgepunktet wird.

4. Verfahren nach Anspruch 1, dadurch gekennzeichnet, daß die Arzneiformträger in Reihe nebeneinander mit dem flüssigen, gelösten oder suspendierten Wirkstoff bepunktet werden.

5. Verfahren nach Anspruch 1, dadurch gekennzeichnet, daß der flüssige, gelöste oder suspendierte Wirkstoff auf das Pulverbett einer noch nicht verschlossenen Kapsel in einer Kapselabfüllmaschine aufgepunktet wird.

6. Verfahren nach Anspruch 1, dadurch gekennzeichnet, daß der flüssige, gelöste oder suspendierte Wirkstoff auf einen flächigen Wirkstoffträger in gewünschten geometrischen Verteilungen aufgepunktet wird.

7. Verfahren nach Anspruch 1 und 6, dadurch gekennzeichnet, daß der flüssige, gelöste oder suspendierte Wirkstoff auf bestimmte Zonen eines flächigen Wirkstoffträgers in voneinander verschiedenen Dosierungen aufgepunktet wird.

8. Verfahren nach Anspruch 1, 6 und 7, dadurch gekennzeichnet, daß der flüssige, gelöste oder suspendierte Wirkstoff mit einem Farbstoff vermischt auf einen Arzneiformträger unter Bildung einer Codierung oder von Schriftzeichen aufgepunktet wird.

## Claims

1. Process for the preparation of solid pharmaceuticals, wherein liquid, dissolved or suspended active substances are divided up, by means of a metering apparatus, into discrete droplets of specific volume and are applied in a specific number to a pharmaceutical carrier, characterised in that the active substances are dotted on by means of piezoelectric metering systems.

2. Process as claimed in claim 1, characterised in that the liquid, dissolved or suspended active substance is dotted into the granulate used as the pharmaceutical carrier before said granulate is compressed to form tablets or cores.

3. Process as claimed in claim 1, characterised in that the liquid, dissolved or suspended active substance is dotted, after the pressing operation, on to the moulding thus obtained.

4. Process as claimed in claim 1, characterised in that the pharmaceutical carriers are dotted, side by side in series, with the liquid, dissolved or suspended active substance.

5. Process as claimed in claim 1, characterised in that the liquid, dissolved or suspended active substance is dotted on to the powder bed of an unsealed capsule in a capsule-filling machine prior to sealing.

6. Process as claimed in claim 1, characterised in that the liquid, dissolved or suspended active substance is dotted on to a flat active-substance carrier in desired geometrical distributions.

7. Process as claimed in claims 1 and 6, characterised in that the liquid, dissolved or suspended active substance ist dotted on to specific zones of a flat active-substance carrier in doses which differ from one another.

8. Process as claimed in claims 1, 6 and 7, characterised in that the liquid, dissolved or suspended active substance is mixed with a dyestuff and dotted on to a pharmaceutical carrier to form a coding or letter characters.

## Revendications

1. Procédé pour fabriquer des médicaments solides, dans lequel des substrances actives liquides, en solution ou en suspension, sont subdivisées en gouttelettes discrètes de volume défini, au moyen d'un dispositif de dosage, et sont déposées en nombre défini sur un support façonné de médicament, caractérisé en ce que les substances actives sont déposées ponctuellement à l'aide de systèmes de dosage piézoélectriques.

2. Procédé suivant la revendication 1, caractérisé en ce que la substance active liquide, en solution ou en suspension, est introduite ponctuellement dans le granulé servant de support façonné de médicament avant que celui-ci soit pressé pour former des comprimés ou des noyaux.

3. Procédé suivant la revendication 1, caractérisé en ce que la substance active liquide, en solution ou en suspension, est déposée ponctuellement après le processus de pressage sur l'ébauche qui en résulte.

4. Procédé suivant la revendication 1, caractérisé en ce que les support façonnés de médicaments sont munis ponctuellement de la substance active liquide, en solution ou en suspension, en série les uns après les autres.

5. Procédé suivant la revendication 1, caractérisé en ce que la substance active liquide, en solution ou en suspension, est déposée ponctuellement sur le lit de poudre d'une capsule non encore fermée dans une machine de remplissage de capsules.

6. Procédé suivant la revendication 1, caractérisé en ce que la substance active liquide, en solution ou en suspension, est déposée ponctuellement sur un support de substance active d'aspect plat suivant des répartitions géométriques souhaitées.

7. Procédé suivant les revendications 1 et 6, caractérisé en ce que la substance active liquide, en solution ou en suspension, est déposée ponctuellement sur des zones déterminées d'un support de substance active d'aspect plat, avec des dosages différents les uns des autres.

8. Procédé suivant les revendications 1, 6 et 7, caractérisé en ce que la substance active liquide, en solution ou en suspension, est mélangée avec un colorant et est déposée ponctuellement sur un support façonné de médicament en formant un code ou des signes graphiques.

0 011 268

FIG.1

FIG.2a

FIG.2b

FIG.2c

9